# EUROPEAN PATENT APPLICATION

(11) **EP 4 792 750 A1**
(43) Date of publication of application: **19.08.2026**
(21) Application number: 23959538.2
(22) Date of filing: 23.11.2023
(51) Int. Cl.: A61B 8/00, A61N 7/02, A61N 7/00

(54) **FOCUSED ULTRASOUND APPARATUS AND NON-INTERFERENCE METHOD BETWEEN ULTRASOUND PULSE SIGNALS**

(30) Priority: 22.11.2023 KR 20230163682
(71) Applicant: IMGT CO., LTD, Gyeonggi-do 13605 (KR)
(72) Inventor: SON, Keon Ho, Seongnam-si Gyeonggi-do 13462 (KR); KOO, Ja Woon, Gimpo-si Gyeonggi-do 10063 (KR); KIM, Dae Seung, Seoul 07524 (KR)
(74) Representative: Sander, Rolf
(86) International application number: PCT/KR2023/018947
(87) International publication number: WO 2025/110281

(57) **Abstract**

Disclosed are a focused ultrasound apparatus and an interference-free method between ultrasound pulse signals. According to one embodiment of the present invention, a focused ultrasound apparatus prevents a treatment ultrasound pulse signal of a treatment module and a diagnosis ultrasound pulse signal of an image module from interfering with each other in a frequency domain and a time domain.

## Description

### [Technical Field]

The present invention relates to diagnosis and treatment technologies using ultrasound signals, and more particularly, to image scanning and treatment technologies using focused ultrasound (FUS) signals for image-guided therapy.

### [Background Art]

Ultrasound may be used to treat biological tissues such as cancer, tumors, and lesions. Treatment using ultrasound is a method of treating a lesion by irradiating the lesion in a human body with an ultrasound pulse signal. Compared with general surgical operations or chemical methods such as chemotherapy, ultrasound treatment causes less trauma to a patient and enables non-invasive treatment. Examples of its application are various, including liver cancer, bone sarcoma, breast cancer, pancreatic cancer, kidney cancer, soft tissue tumor, and pelvic tumor.

### [Disclosure]

### [Technical problem]

According to an embodiment, provided are a focused ultrasound apparatus and a non-interference method between ultrasound pulse signals, which prevent a treatment ultrasound pulse signal of a treatment module and a diagnosis ultrasound pulse signal of an imaging module from interfering with each other.

### [Technical Solution]

According to an embodiment, a focused ultrasound apparatus includes a treatment module configured to receive information on parameters of a treatment ultrasound pulse signal (hereinafter, referred to as "treatment ultrasound parameters") and generate the treatment ultrasound pulse signal according to the received treatment ultrasound parameter information, and an imaging module configured to receive the treatment ultrasound parameter information and information on parameters of a diagnosis ultrasound pulse signal (hereinafter, referred to as "diagnosis ultrasound parameters"), analyze the received treatment ultrasound parameter information and diagnosis ultrasound parameter information in a frequency domain, adjust the diagnosis ultrasound parameter information such that the treatment ultrasound pulse signal and the diagnosis ultrasound pulse signal do not interfere with each other, and generate the diagnosis ultrasound pulse signal according to the adjusted diagnosis ultrasound parameter information.

The imaging module may shift a frequency of the diagnosis ultrasound pulse signal (hereinafter, referred to as a "diagnosis ultrasound frequency") such that a frequency of the treatment ultrasound pulse signal (hereinafter, referred to as a "treatment ultrasound frequency") and the diagnosis ultrasound frequency do not overlap each other in a frequency domain, and may generate a new diagnosis ultrasound frequency by shifting the frequency.

The shifted new diagnosis ultrasound frequency may be greater than a sum of the treatment ultrasound frequency and a bandwidth of the diagnosis ultrasound pulse signal (hereinafter, referred to as a "diagnosis ultrasound bandwidth").

The imaging module may include a diagnosis ultrasound pulse generator configured to receive diagnosis ultrasound parameter information including information on a center frequency of a diagnosis ultrasound pulse signal (hereinafter, referred to as a "diagnosis ultrasound center frequency") (Fc_img) and information on a diagnosis ultrasound bandwidth (Fc_img B.W), receive treatment ultrasound parameter information including information on a center frequency of a treatment ultrasound pulse signal (hereinafter, referred to as a "treatment ultrasound center frequency") (Fc_fus), and adjust the diagnosis ultrasound parameter information for non-interference between the treatment ultrasound pulse signal and the diagnosis ultrasound pulse signal by using the diagnosis ultrasound parameter information and the treatment ultrasound parameter information.

The diagnosis ultrasound pulse generator may shift the diagnosis ultrasound center frequency (Fc_img) to generate a new diagnosis ultrasound center frequency (Fc'_img) such that the new diagnosis ultrasound center frequency (Fc'_img) is greater than a sum of the treatment ultrasound center frequency (Fc_fus) and the diagnosis ultrasound bandwidth (Fc_img B.W) in a frequency domain.

The imaging module may include an image processing unit configured to generate a diagnosis image by receiving an ultrasound echo signal in an interval in which the frequency of the treatment ultrasound frequency and the frequency of the diagnosis ultrasound frequency do not overlap.

The treatment module may acquire timing information regarding an interval in which the treatment ultrasound pulse signal is actually generated in a time domain, and the imaging module may receive the timing information from the treatment module and generate the diagnosis ultrasound pulse signal during an interval in which the treatment ultrasound pulse signal is not generated.

The treatment module may include a signal extraction unit configured to extract an active period of the treatment ultrasound pulse signal generated in each channel of a treatment ultrasound transducer, and a summation unit configured to obtain an overall active period of the treatment ultrasound pulse signals by performing a logical OR operation on the active periods of the treatment ultrasound pulse signals of the respective channels.

The signal extraction unit may include an attenuator configured to attenuate the treatment ultrasound pulse signal generated in each channel, and an envelope detector configured to extract an envelope of each of the attenuated treatment ultrasound pulse signals.

The summation unit may transmit an image pulse activation signal to the imaging module during an interval excluding an overall treatment ultrasound active period, and the imaging module may generate the diagnosis ultrasound pulse signal during an interval excluding the overall treatment ultrasound active period according to the image pulse activation signal.

The focused ultrasound apparatus may further include a comparator configured to receive, as inputs, a signal extracted through the signal extraction unit and a reference signal, compare the extracted signal and the reference signal with each other, and output the extracted signal as a logic signal when the extracted signal has a value equal to or greater than a value of the reference signal, and a reference providing unit configured to provide the reference signal to the comparator.

The reference providing unit may be a digital-to-analog converter capable of varying the value of the reference signal.

According to another embodiment, a non-interference method between ultrasound pulse signals using a focused ultrasound apparatus includes receiving, by an imaging module of the focused ultrasound apparatus, treatment ultrasound parameter information and diagnosis ultrasound parameter information, adjusting the diagnosis ultrasound parameter information such that a treatment ultrasound pulse signal and a diagnosis ultrasound pulse signal do not interfere with each other by analyzing the received treatment ultrasound parameter information and diagnosis ultrasound parameter information in a frequency domain, generating the diagnosis ultrasound pulse signal according to the adjusted diagnosis ultrasound parameter information and transmitting the generated diagnosis ultrasound pulse signal to tissue, and generating a diagnosis image using an ultrasound echo signal returned from the tissue.

### [Advantages and Effects]

According to a focused ultrasound apparatus and a non-interference method between ultrasound pulse signals according to an embodiment, the apparatus may be operated such that a treatment ultrasound pulse signal of a treatment module does not interfere with a diagnosis ultrasound pulse signal of an imaging module in a frequency domain and a time domain, and a diagnosis image may be obtained while performing a treatment operation using the treatment ultrasound pulse signal, thereby allowing a user to easily perform a procedure.

In the case of an interleaving method in which a diagnosis ultrasound output time and a treatment ultrasound output time are scheduled for non-interference between two signals in the time domain, a separate timing controller is required and control is difficult. However, according to the focused ultrasound apparatus and the non-interference method between ultrasound pulse signals according to an embodiment, a separate timing controller is unnecessary, making control simple and economical.

### [Description of Drawings]

FIG. 1 is a diagram illustrating an example in which an artifact is generated in a diagnosis image due to interference between a diagnosis ultrasound pulse signal and a treatment ultrasound pulse signal according to an embodiment of the present invention.
FIG. 2 is a block diagram illustrating a configuration of a focused ultrasound apparatus according to an embodiment of the present invention.
FIG. 3 is a diagram illustrating a configuration of a focused ultrasound apparatus for non-interference between two ultrasound pulse signals in a frequency domain according to an embodiment of the present invention.
FIG. 4 is a diagram illustrating an example in which interference between a diagnosis ultrasound pulse signal and a treatment ultrasound pulse signal is removed by a diagnosis ultrasound frequency shift according to an embodiment of the present invention.
FIG. 5 is a diagram illustrating a configuration of a focused ultrasound apparatus for non-interference between ultrasound pulse signals in a time domain according to an embodiment of the present invention.
FIG. 6 is a diagram illustrating a configuration of a focused ultrasound apparatus for explaining a non-interference operation between ultrasound pulse signals according to an embodiment of the present invention.
FIG. 7 is a diagram illustrating a configuration of an attenuator according to an embodiment of the present invention.
FIG. 8 is a diagram illustrating a configuration of an envelope detector according to an embodiment of the present invention.
FIG. 9 is a diagram illustrating a configuration of a comparator according to an embodiment of the present invention.
FIG. 10 is a diagram illustrating waveforms of signals generated over time by a focused ultrasound apparatus including n channels according to an embodiment of the present invention.
FIG. 11 is a diagram illustrating waveforms of signals generated for non-interference between ultrasound pulse signals of a focused ultrasound apparatus according to an embodiment of the present invention.
FIG. 12 is a flowchart illustrating a non-interference method between ultrasound pulse signals in a frequency domain according to an embodiment of the present invention.
FIG. 13 is a flowchart illustrating a non-interference method between ultrasound pulse signals in a time domain according to an embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. The same reference numerals refer to the same components throughout this disclosure.

In the following description of the embodiments of the present invention, if a detailed description of related known functions or configurations is determined to unnecessarily obscure the gist of the present invention, the detailed description thereof will be omitted herein. The terms described below are defined in consideration of the functions in the embodiments of the present invention, and these terms may be varied according to the intent or custom of a user or an operator. Therefore, the definitions of the terms used herein should follow contexts disclosed herein.

It will be understood that each block of the flowcharts and/or block diagrams, and combinations of blocks in the flowcharts and/or block diagrams, can be implemented by computer program instructions (execution engine). These computer program instructions may be provided to a processor of a general-purpose computer, special purpose computer, or other programmable data processing apparatus, such that the instructions which are executed via the processor of the computer or other programmable data processing apparatus create means for implementing the functions/acts specified in the flowcharts and/or block diagrams.

These computer program instructions may also be stored in a non-transitory computer-readable memory that can direct a computer or other programmable data processing apparatus to function in a particular manner, such that the instructions stored in the non-transitory computer-readable memory produce articles of manufacture embedding instruction means which implement the function/act specified in the flowcharts and/or block diagrams.

The computer program instructions may also be loaded onto a computer or other programmable data processing apparatus to cause a series of operations to be performed on the computer or other programmable apparatus to produce a computer implemented process such that the instructions which are executed on the computer or other programmable apparatus provide operations for implementing the functions/acts specified in the flowcharts and/or block diagrams.

Furthermore, the respective blocks of the block diagrams and/or flowcharts may illustrate parts of modules, segments, or codes including at least one or more executable instructions for performing specific logic function(s). Moreover, it should be noted that the functions of the blocks may be performed in a different order in several modifications. For example, two successive blocks may be performed substantially at the same time, or may be performed in reverse order according to their functions.

Hereinafter, embodiments of the present invention will be described in detail with reference to the accompanying drawings. However, the present invention may be realized in various forms, and the scope of the present invention is not limited to such embodiments.
The embodiments of the present invention are provided to aid those skilled in the art in the explanation and the understanding of the present invention.

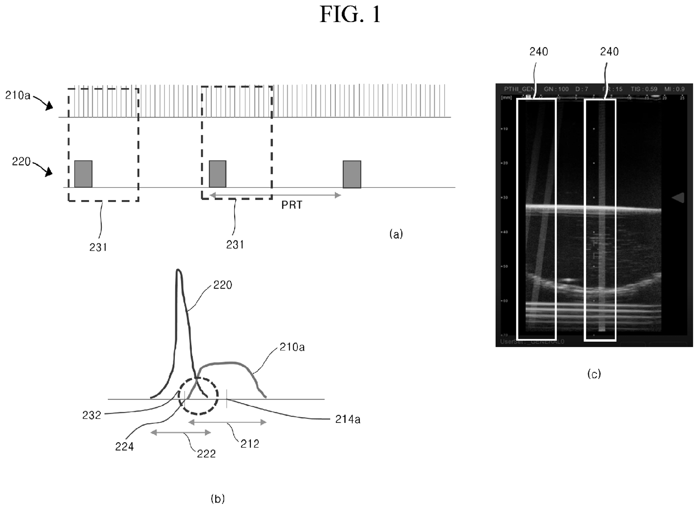

FIG. 1 is a diagram illustrating an example in which an artifact is generated in a diagnosis image due to interference between a diagnosis ultrasound pulse signal and a treatment ultrasound pulse signal according to an embodiment of the present invention.

More specifically, (a) is a diagram illustrating an example in which interference occurs between a diagnosis ultrasound pulse signal and a treatment ultrasound pulse signal in a time domain, (b) is a diagram illustrating an example in which interference occurs between the diagnosis ultrasound pulse signal and the treatment ultrasound pulse signal in a frequency domain, and (c) is a diagram illustrating an example of a diagnosis image in which artifacts are generated when interference occurs between the two signals.

Referring to FIG. 1, as shown in (a) and (b), when a diagnosis ultrasound pulse signal 210a and a treatment ultrasound pulse signal 220 are transmitted, interference 231 occurs between the two signals 210a and 220.

In a time domain of (a) of FIG. 1, the diagnosis ultrasound pulse signal 210a is a short pulse signal having a center frequency Fc_img (hereinafter, referred to as a "diagnosis ultrasound center frequency") of 3.5 MHz, a pulse repetition time (PRT) of 250 us, and a Vpp of 150 V. The treatment ultrasound pulse signal 220 is a long pulse signal having a center frequency Fc_fus (hereinafter, referred to as a "treatment ultrasound center frequency") of 1 MHz, a PRT of 1,000 us, a Vpp of 300 V, and an output time of 5 ms. When the diagnosis ultrasound pulse signal 210a and the treatment ultrasound pulse signal 220 are transmitted, it can be confirmed that the interference 231 occurs between the two signals 210a and 220 in the time domain.

Interference 232 also occurs between the two signals 210a and 220 in a frequency domain of (b) of FIG. 1. The diagnosis ultrasound center frequency Fc_img is 3.5 MHz, and reference numeral 212 denotes a bandwidth of the diagnosis ultrasound pulse signal 210a (hereinafter, referred to as a "diagnosis ultrasound bandwidth").

The treatment ultrasound pulse center frequency Fc_fus is 1.0 MHz, and reference numeral 222 denotes a bandwidth of the treatment ultrasound pulse signal 220 (hereinafter, referred to as a "treatment ultrasound bandwidth").

When the diagnosis ultrasound pulse signal 210a and the treatment ultrasound pulse signal 220 are transmitted, it can be confirmed that the interference 232 occurs between the two signals 210a and 220 in the frequency domain.

Due to such interference 231 and 232 between the two signals in the time domain and the frequency domain, a severe artifact 240 pattern is detected in the diagnosis image, as shown in (c). An artifact is undesired noise.

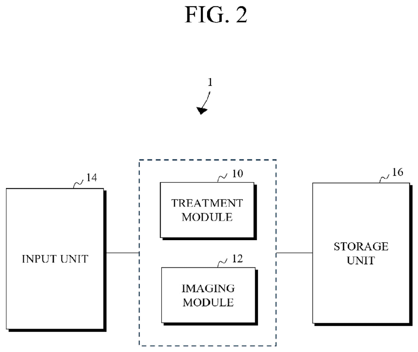

FIG. 2 is a block diagram illustrating a configuration of a focused ultrasound apparatus according to an embodiment of the present invention.

Referring to FIG. 2, a focused ultrasound apparatus 1 includes a treatment module 10, an imaging module 12, an input unit 14, and a storage unit 16.

The focused ultrasound apparatus 1 according to an embodiment transmits a treatment ultrasound pulse signal to tissue through the treatment module 10 for treatment. The treatment ultrasound pulse signal is a focused ultrasound (FUS) signal. The focused ultrasound apparatus 1 transmits a diagnosis ultrasound pulse signal to tissue in order to obtain a diagnosis image. In this case, the focused ultrasound apparatus 1 may obtain a diagnosis image using the diagnosis ultrasound pulse signal of the imaging module 12 while simultaneously performing a treatment operation using the treatment ultrasound pulse signal.

The treatment module 10 and the imaging module 12 include a processor. The processor controls the overall operation of each component of the focused ultrasound apparatus 1. The processor may perform operations on at least one application or program for executing methods/operations according to various embodiments of the present disclosure.

The input unit 14 receives, according to a user manipulation signal, parameters of the treatment ultrasound pulse signal (hereinafter, referred to as "treatment ultrasound parameters") and parameters of the diagnosis ultrasound pulse signal (hereinafter, referred to as "diagnosis ultrasound parameters"). In this case, each parameter may be an output time, a frequency, an acoustic pressure, an intensity, a waveform, a duty cycle, or the like of the corresponding signal. Frequency information may include center frequency fc and bandwidth information.

The storage unit 16 stores various types of data, commands, and/or information. The storage unit 16 may load one or more computer programs in order to execute methods/operations according to various embodiments of the present disclosure. When a computer program is loaded in the storage unit 16, the processor may perform methods/operations according to various embodiments of the present disclosure by executing one or more instructions.

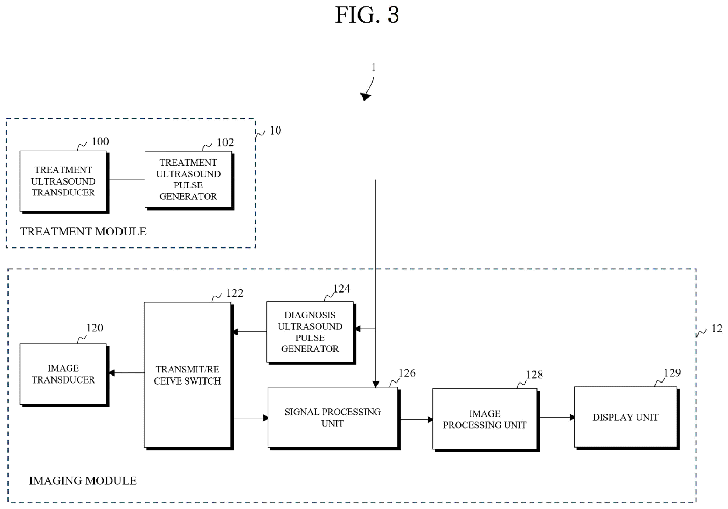

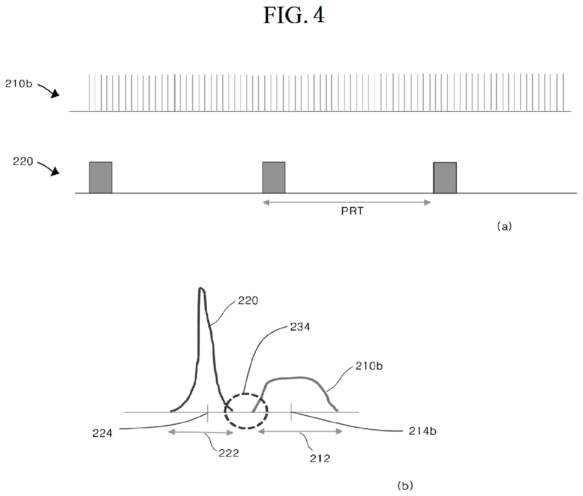

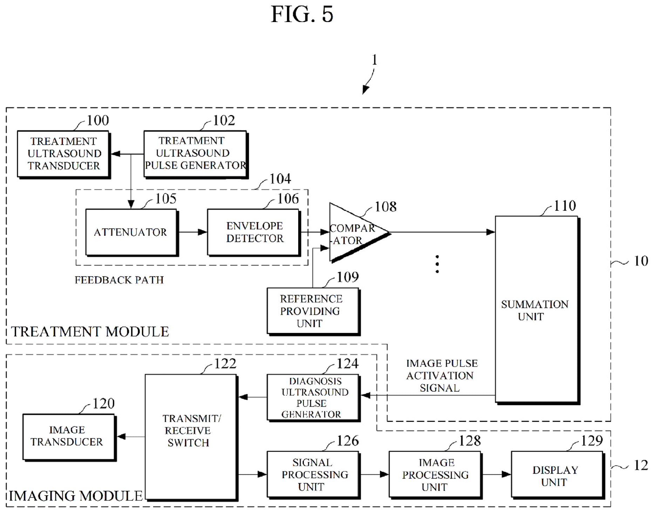

The focused ultrasound apparatus 1 according to an embodiment is an apparatus for non-interference between a treatment ultrasound pulse signal and a diagnosis ultrasound pulse signal in a frequency domain or a time domain. Hereinafter, configurations and embodiments for non-interference between the two signals in the frequency domain will be described with reference to FIGS. 3 and 4, and configurations and embodiments for non-interference between the two signals in the time domain will be described with reference to FIGS. 5 to 11.

FIG. 3 is a diagram illustrating a configuration of a focused ultrasound apparatus for non-interference between two ultrasound pulse signals in a frequency domain according to an embodiment of the present invention.

Referring to FIGS. 2 and 3, the focused ultrasound apparatus 1 includes a treatment module 10, an imaging module 12, an input unit 14, and a storage unit 16.

The treatment module 10 includes a treatment ultrasound transducer 100 and a treatment ultrasound pulse generator 102.

The imaging module 12 includes an image transducer 120, a transmit/receive switch 122, a diagnosis ultrasound pulse generator 124, a signal processing unit 126, an image processing unit 128, and a display unit 129.

The treatment ultrasound pulse generator 102 receives, from the input unit 14, treatment ultrasound parameter information including treatment ultrasound center frequency Fc_fus information and treatment ultrasound bandwidth Fc_fus B.W, generates a treatment ultrasound pulse signal having the received treatment ultrasound center frequency Fc_fus and treatment ultrasound bandwidth Fc_fus B.W, and transmits the generated treatment ultrasound pulse signal to the treatment ultrasound transducer 100.

The treatment ultrasound transducer 100 transmits the treatment ultrasound pulse signal received from the treatment ultrasound pulse generator 102 to tissue. The treatment ultrasound pulse signal causes a local tissue temperature rise through transmission of ultrasound energy within the tissue. The treatment ultrasound pulse signal may be a focused ultrasound signal. The focused ultrasound signal may be a high-intensity focused ultrasound (HIFU) signal.

The diagnosis ultrasound pulse generator 124 receives, from the input unit 14, diagnosis ultrasound parameter information, for example, diagnosis ultrasound center frequency Fc_img information and diagnosis ultrasound bandwidth Fc_img B.W information, and receives, from the treatment ultrasound pulse generator 102, treatment ultrasound parameter information, for example, treatment ultrasound center frequency Fc_fus information.

Subsequently, the diagnosis ultrasound pulse generator 124 analyzes the diagnosis ultrasound parameter information and the treatment ultrasound parameter information, and adjusts the diagnosis ultrasound parameter information for non-interference between the treatment ultrasound pulse signal and the diagnosis ultrasound pulse signal. For example, in the frequency domain, the diagnosis ultrasound pulse generator 124 shifts the diagnosis ultrasound center frequency Fc_img to generate a new diagnosis ultrasound center frequency Fc'_img such that the new diagnosis ultrasound center frequency Fc'_img is greater than a sum of the treatment ultrasound center frequency Fc_fus and the diagnosis ultrasound bandwidth Fc_img B.W. That is, Fc'_img > Fc_fus + Fc_img B.W.

The diagnosis ultrasound pulse generator 124 generates a diagnosis ultrasound pulse signal having the new diagnosis ultrasound center frequency Fc'_img and transmits the generated diagnosis ultrasound pulse signal to the image transducer 120.

The image transducer 120 transmits the diagnosis ultrasound pulse signal having the new diagnosis ultrasound center frequency Fc'_img into tissue, and receives an ultrasound echo signal reflected from the tissue. The image transducer 120 receives the ultrasound echo signal until the next diagnosis ultrasound pulse signal is generated, and transmits the received ultrasound echo signal to the transmit/receive switch 122.

The transmit/receive switch 122 switches between a transmit mode and a receive mode of the imaging module 12, and is electrically connected to the image transducer 120 in the receive mode to receive the ultrasound echo signal.

The signal processing unit 126 processes the received ultrasound echo signal. For example, the signal processing unit 126 may include a low noise amplifier (LNA) and an analog-to-digital (A/D) converter (ADC). The low noise amplifier receives the ultrasound echo signal from the image transducer 120 via the transmit/receive switch 122, and amplifies the received ultrasound echo signal with a minimum noise level. The A/D converter converts the ultrasound echo signal output from the low noise amplifier into a digital signal, and transmits the digital signal to the image processing unit 128. The imaging module 12 outputs a diagnosis image through the display unit 129 after image processing through the image processing unit 128.

The image processing unit 128 may generate the diagnosis image by receiving an ultrasound echo signal in an interval in which the diagnosis ultrasound frequency and the treatment ultrasound frequency do not overlap. To this end, the image processing unit 128 may include a high-pass filter (HPF). A high-pass filter is used to remove or suppress low-frequency components. The high-pass filter may be applied to sharply process an image. Accordingly, fine boundary details are preserved.

FIG. 4 is a diagram illustrating an example in which interference between a diagnosis ultrasound pulse signal and a treatment ultrasound pulse signal is removed by a diagnosis ultrasound frequency shift according to an embodiment of the present invention.

More specifically, (a) is a diagram illustrating an example in which a diagnosis ultrasound frequency is shifted in order to remove interference between a diagnosis ultrasound pulse signal and a treatment ultrasound pulse signal in a time domain, and (b) is a diagram illustrating an example in which the diagnosis ultrasound frequency is shifted in order to remove interference between the diagnosis ultrasound pulse signal and the treatment ultrasound pulse signal in a frequency domain.

Referring to FIGS. 1, 3, and 4, the signal processing unit 126 shifts the diagnosis ultrasound center frequency Fc_img 214a to generate a new diagnosis ultrasound center frequency Fc'_img 214b such that the new diagnosis ultrasound center frequency Fc'_img 214b is greater than a sum of the treatment ultrasound center frequency Fc_fus 224 and the diagnosis ultrasound bandwidth Fc_img B.W 212.

For example, when the treatment ultrasound center frequency Fc_fus 224 of the treatment ultrasound pulse signal 220 is 1.0 MHz, the diagnosis ultrasound bandwidth Fc_img B.W 212 is 4 MHz, and the diagnosis ultrasound center frequency Fc_img 214a is 1 MHz, Fc'_img > Fc_fus (1 MHz) + Fc_img B.W (4 MHz). Accordingly, the new diagnosis ultrasound center frequency Fc'_img 214b of the new diagnosis ultrasound pulse signal 210b is greater than 5 MHz. For example, the new diagnosis ultrasound center frequency Fc'_img 214b is 5.5 MHz.

The image processing unit 128 may generate a diagnosis image by receiving an ultrasound echo signal in an interval 234 in which the diagnosis ultrasound frequency and the treatment ultrasound frequency do not overlap in the frequency domain. To this end, the image processing unit 128 may include a high-pass filter (HPF).

FIG. 5 is a diagram illustrating a configuration of a focused ultrasound apparatus for non-interference between ultrasound pulse signals in a time domain according to an embodiment of the present invention.

Referring to FIGS. 2 and 5, the focused ultrasound apparatus 1 according to an embodiment analyzes treatment ultrasound parameters such that a treatment ultrasound pulse signal does not interfere with a diagnosis ultrasound pulse signal, and in this case, a separate timing controller is unnecessary. To this end, the focused ultrasound apparatus 1 acquires, in real-time, timing information on an interval during which the treatment ultrasound pulse signal is actually generated through the treatment module 10 and transmits the timing information to the imaging module 12, and the imaging module 12 receives the timing information from the treatment module 10 and generates a diagnosis ultrasound pulse signal sequence during an interval during which the treatment ultrasound pulse signal is not generated. Accordingly, a separate timing controller is not required, thereby simplifying control.

Referring to FIG. 5, the treatment module 10 may include a treatment ultrasound transducer 100, a treatment ultrasound pulse generator 102, a signal extraction unit 104, a comparator 108, a reference providing unit 109, and a summation unit 110. The signal extraction unit 104 may include an attenuator 105 and an envelope detector 106.

The imaging module 12 may include an image transducer 120, a transmit/receive switch 122, a diagnosis ultrasound pulse generator 124, a signal processing unit 126, an image processing unit 128, and a display unit 129.

The treatment ultrasound pulse generator 102 generates a treatment ultrasound pulse signal using treatment ultrasound parameter information and then transmits the generated treatment ultrasound pulse signal to the treatment ultrasound transducer 100.

The treatment ultrasound transducer 100 transmits the treatment ultrasound pulse signal received from the treatment ultrasound pulse generator 102 to tissue so as to cause a local tissue temperature rise through transmission of ultrasound energy within the tissue. The treatment ultrasound pulse signal may be a high-intensity focused ultrasound (HIFU) signal.

The image transducer 120 transmits the diagnosis ultrasound pulse signal received from the diagnosis ultrasound pulse generator 124 into tissue, and receives an ultrasound echo signal reflected from the tissue. The image transducer 120 receives the ultrasound echo signal until the next diagnosis ultrasound pulse signal is generated, and transmits the received ultrasound echo signal to the transmit/receive switch 122.

The focused ultrasound apparatus 1 may have a structure in which the image transducer 120 is positioned at a center and the treatment ultrasound transducer 100 is arranged in a peripheral portion. However, the structures of the image transducer 120 and the treatment ultrasound transducer 100 are not limited thereto, and may be variously modified.

The signal extraction unit 104 extracts an active period of the treatment ultrasound pulse signal generated in each channel of the treatment ultrasound transducer 100. The signal extraction unit 104 may include the attenuator 105 and the envelope detector 106. The attenuator 105 attenuates the treatment ultrasound pulse signal generated in each channel without a change in impedance, and the envelope detector 106 extracts an envelope of the treatment ultrasound pulse signal attenuated through the attenuator 105. The signal extraction unit 104 may be replaced with a low-pass filter (LPF).

The summation unit 110 obtains an overall treatment ultrasound active period of the treatment ultrasound pulse signals by performing a logical OR operation on the active periods of the treatment ultrasound pulse signals of the respective channels. The summation unit 110 transmits an image pulse activation signal to the imaging module 12 during an interval excluding the overall treatment ultrasound active period, and the diagnosis ultrasound pulse generator 124 of the imaging module 12 may generate the diagnosis ultrasound pulse signal during an interval excluding the overall treatment ultrasound active period according to the image pulse activation signal.

The treatment module 10 according to an embodiment may further include a comparator 108 and a reference providing unit 109. The treatment module 10 attenuates a signal in each individual channel by using the attenuator 105 without a change in impedance, obtains an active period of each channel by using the envelope detector 106 and the comparator 108, obtains an overall treatment ultrasound active period by performing a logical OR operation on the active periods of all channels through the summation unit 110, and then transmits timing information of the active period to the imaging module 12. In the imaging module 12, an image pulse sequence is generated during an interval in which the treatment ultrasound pulse signal is not activated.

The comparator 108 receives, as respective inputs, the signal extracted through the signal extraction unit 104 and a reference signal, compares the extracted signal with the reference signal, and outputs the extracted signal as a logic signal if the extracted signal has a value equal to or greater than the value of the reference signal. The reference providing unit 109 provides the reference signal to the comparator 108. For example, a reference value of 0.4 V is provided to the comparator 108, and the comparator 108 outputs logic signal 1 when the signal extracted through the signal extraction unit 104 is 0.4 V or greater. The reference providing unit 109 may be a digital-to-analog converter capable of varying the value of the reference signal.

The attenuator 105, the envelope detector 106, the comparator 108, the reference providing unit 109, and the summation unit 110 are formed in a feedback path.

The transmit/receive switch 122 of the imaging module 12 switches between a transmit mode and a receive mode of the imaging module 12, and is electrically connected to the image transducer 120 in the receive mode to receive the ultrasound echo signal.

The signal processing unit 126 processes the received ultrasound echo signal. For example, the signal processing unit 126 may include a low noise amplifier (LNA) and an analog-to-digital (A/D) converter (ADC). The low noise amplifier receives the ultrasound echo signal from the image transducer 120 via the transmit/receive switch 122, and amplifies the received signal with a minimum noise level. The A/D converter converts the ultrasound echo signal output from the low noise amplifier into a digital signal, and transmits the digital signal to the image processing unit 128. The imaging module 12 outputs a diagnosis image through the display unit 129 after image processing through the image processing unit 128.

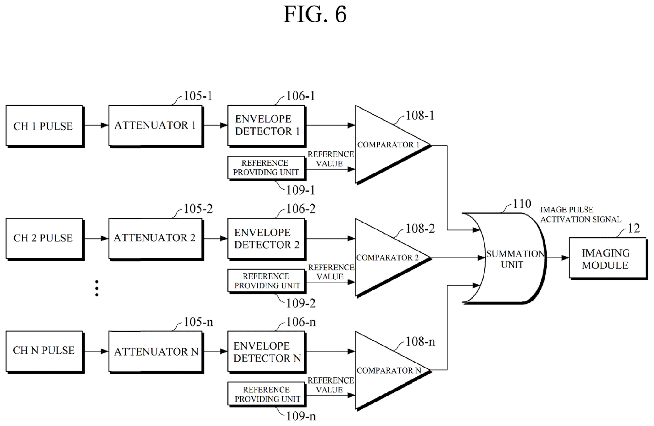

FIG. 6 is a diagram illustrating a configuration of a focused ultrasound apparatus for explaining a non-interference operation between ultrasound pulse signals according to an embodiment of the present invention.

Referring to FIGS. 5 and 6, when the treatment module 10 has n channels CH, signals in the individual channels 1 to n are attenuated by using attenuators 105-1 to 105-n, respectively, without a change in impedance, and active periods of the respective channels are obtained by using envelope detectors 106-1 to 106-n and comparators 108-1 to 108-n, respectively.

Although n reference providing units 109-1 to 109-n are illustrated in FIG. 6, only a single reference providing unit 109 may be used when values of the reference signals in the respective channels are identical.

Subsequently, the treatment module 10 performs a logical OR operation on the active periods of all channels through the summation unit 110 to obtain an overall active period, and transmits the obtained logical OR value to the imaging module 12. In the imaging module 12, an image pulse sequence is executed during an interval during which the treatment ultrasound pulse signal is not activated.

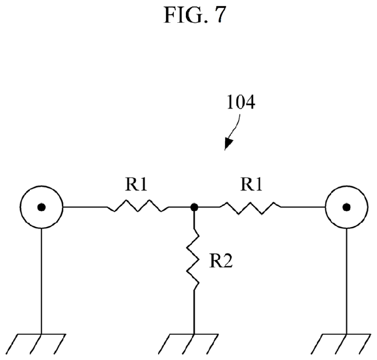

FIG. 7 is a diagram illustrating a configuration of an attenuator according to an embodiment of the present invention.

Referring to FIGS. 5 and 7, since the treatment module 10 constitutes a feedback path for a high-voltage signal, the attenuator 105 is to be configured in order to attenuate a level of the signal without affecting output impedance matching.

As shown in FIG. 7, the attenuator 105 may be configured in a T shape, and has resistance values R1 and R2 satisfying the following equations. $R 1 = Z ⋅ \frac{K - 1}{K + 1}$ $R 2 = 2 Z ⋅ \frac{K}{{K}^{2} - 1}$

Here, K is 10^{α/20}, and α is the amount of attenuation (dB).

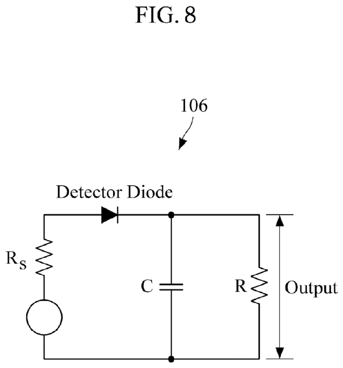

FIG. 8 is a diagram illustrating a configuration of an envelope detector according to an embodiment of the present invention.

Referring to FIGS. 5 and 8, the envelope detector 106 includes a detector diode, a capacitor C, and a resistor R.

The envelope detector 106 is generally used as an input FOR a communication amplitude modulation (AM) demodulator, but here is used to generate an active period of a pulse output by using the attenuated treatment ultrasound pulse signal as an input.

When passing through the detector diode, a negative region of the signal is removed, a signal in a positive interval is charged in the capacitor C, and an envelope signal appears across the resistor R.

An RC time constant should be sufficiently long so that the envelope signal can be maintained, and should satisfy the following equation. $1 / Fc ≪ RC ≪ 1 / W$

Here, Fc is a carrier frequency, and W is a maximum modulation frequency (maximum FUS PRF).

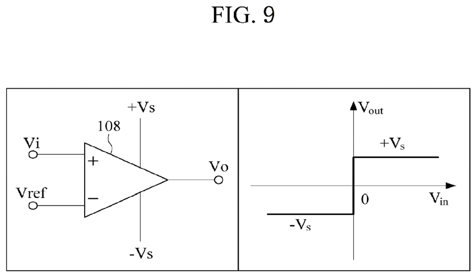

FIG. 9 is a diagram illustrating a configuration of a comparator according to an embodiment of the present invention.

Referring to FIGS. 5 and 9, since the signal passing through the envelope detector 106 is an attenuated analog signal of a high-voltage pulse, the analog signal should be converted into a logic signal (TTL, CMOS) so as to be provided as an input for a logical OR operation of the summation unit 110. When a voltage Vi equal to or greater than a predetermined reference voltage Vref is input through the comparator 108, logic 1 (HIGH) signal may be output.

As shown in FIG. 9, the comparator 108 may be configured as an operational amplifier (OP amp) capable of outputting logic 1 when a value greater than the reference voltage Vref is input, and the reference providing unit 109 may be a DAC so that the value of the reference voltage Vref can be varied.

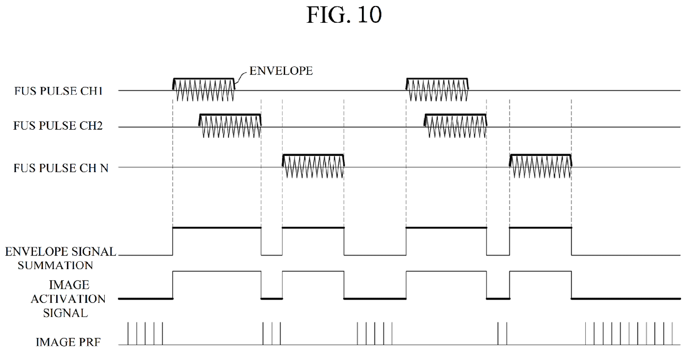

FIG. 10 is a diagram illustrating waveforms of signals generated over time by a focused ultrasound apparatus including n channels according to an embodiment of the present invention.

Referring to FIGS. 5 and 10, the treatment module 10 extracts an envelope signal from each of treatment ultrasound channels CH1 to CH N constituting n channels.

Subsequently, the treatment module 10 obtains an overall treatment ultrasound active period by performing a logical OR operation on the respective envelope signals through the summation unit 110, and transmits, to the imaging module 12, an image pulse activation signal for causing a diagnosis ultrasound pulse signal to be generated during an interval excluding the overall treatment ultrasound active period. In this case, the imaging module 12 receives the image pulse activation signal and generates the diagnosis ultrasound pulse signal only during an interval in which the treatment ultrasound is not operating.

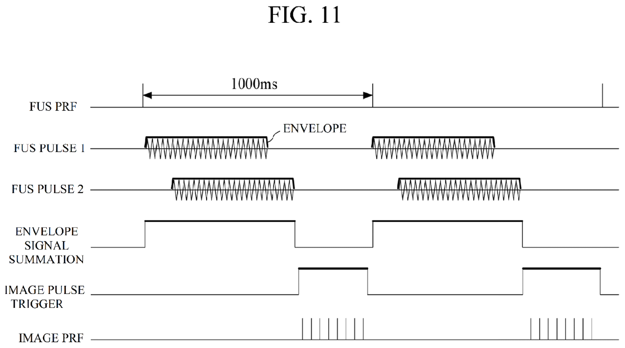

FIG. 11 is a diagram illustrating waveforms of signals generated for non-interference between ultrasound pulse signals of a focused ultrasound apparatus according to an embodiment of the present invention.

Referring to FIGS. 5 and 11, when the treatment module 10 generates treatment ultrasound pulse signals having a predetermined PRT, for example, 1,000 ms, envelope signals of treatment ultrasound pulse signal 1 and treatment ultrasound pulse signal 2 are respectively obtained through the envelope detector 106, and then a logical OR operation is performed on the envelope signal of treatment ultrasound pulse signal 1 and the envelope signal of treatment ultrasound pulse signal 2 through the summation unit 110. Subsequently, the treatment module 10 generates an image pulse activation signal during an interval excluding the overall active period of the treatment ultrasound pulse signals obtained through the logical OR operation and transmits the image pulse activation signal to the imaging module 12. The imaging module 12 generates an image pulse sequence during an interval excluding the overall active period of the treatment ultrasound pulse signals according to the image pulse activation signal.

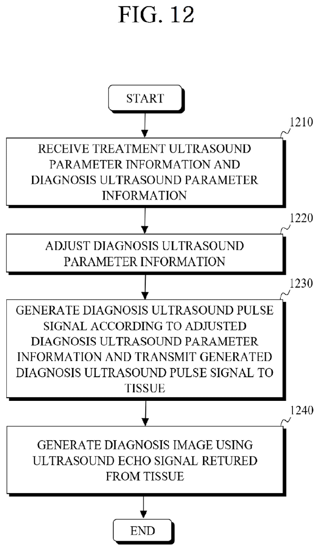

FIG. 12 is a flowchart illustrating a non-interference method between ultrasound pulse signals in a frequency domain according to an embodiment of the present invention.

Referring to FIGS. 3 and 12, the imaging module 12 receives treatment ultrasound parameter information and diagnosis ultrasound parameter information (1210). In this case, the imaging module 12 may receive diagnosis ultrasound parameter information including diagnosis ultrasound center frequency Fc_img information and diagnosis ultrasound bandwidth Fc_img B.W information, and treatment ultrasound parameter information including treatment ultrasound center frequency Fc_fus information.

Subsequently, the imaging module 12 analyzes the received treatment ultrasound parameter information and diagnosis ultrasound parameter information in a frequency domain and adjusts the diagnosis ultrasound parameter information such that the treatment ultrasound pulse signal and the diagnosis ultrasound pulse signal do not interfere with each other (1220). For example, in the frequency domain, the imaging module 12 may shift the diagnosis ultrasound center frequency Fc_img to generate a new diagnosis ultrasound center frequency Fc'_img such that the new diagnosis ultrasound center frequency Fc'_img is greater than a sum of the treatment ultrasound center frequency Fc_fus and the diagnosis ultrasound bandwidth Fc_img B.W.

Subsequently, the imaging module 12 generates a diagnosis ultrasound pulse signal according to the adjusted diagnosis ultrasound parameter information and transmits the generated diagnosis ultrasound pulse signal to tissue (1230).

Subsequently, the imaging module 12 generates a diagnosis image by using an ultrasound echo signal returned from the tissue (1240).

While the imaging module 12 transmits a new diagnosis ultrasound pulse signal having the new diagnosis ultrasound center frequency Fc'_img, the treatment module 10 transmits a treatment ultrasound pulse signal having the treatment ultrasound center frequency. In this case, the new diagnosis ultrasound pulse signal and the treatment ultrasound pulse signal do not interfere with each other.

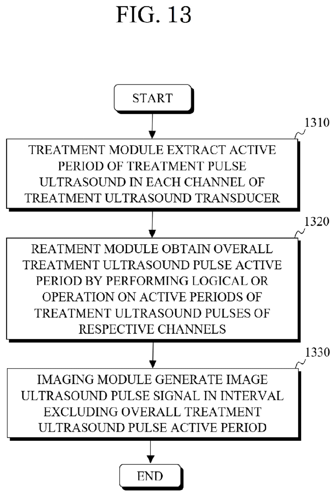

FIG. 13 is a flowchart illustrating a non-interference method between ultrasound pulse signals in a time domain according to an embodiment of the present invention.

Referring to FIGS. 5 and 13, the treatment module 10 extracts an active period of a treatment ultrasound pulse generated in each channel of the treatment ultrasound transducer 100 (1310).

Subsequently, the treatment module 10 obtains an overall treatment ultrasound pulse active period by performing a logical OR operation on the active periods of the treatment ultrasound pulses of the respective channels (1320).

Subsequently, the imaging module 12 generates an image ultrasound pulse during an interval excluding the overall treatment ultrasound pulse active period (1330).

In operation 1310 of extracting the active period of the treatment ultrasound pulse, the treatment module 10 may attenuate the treatment ultrasound pulse generated in each channel and then extract an envelope of each attenuated treatment ultrasound pulse. Furthermore, after a comparator receives, as respective inputs, each extracted envelope signal and a reference signal and compares them with each other, the treatment module 10 may output a logic signal when the extracted signal has a value equal to or greater than the value of the reference signal.

Furthermore, the method may further include transmitting, by the treatment module 10, an image pulse activation signal to the imaging module 12 during an interval excluding an overall treatment ultrasound active period, and in operation 1330 of generating the image ultrasound pulse, the imaging module 12 may generate the image ultrasound pulse during an interval excluding the overall treatment ultrasound active period according to the image pulse activation signal.

The present invention has been described above with reference to embodiments thereof. Those skilled in the art to which the present invention pertains will understand that the present invention may be implemented in a modified form without departing from the essential characteristics of the present invention. Therefore, the disclosed embodiments should be considered in a descriptive sense rather than a restrictive sense. The scope of the present invention is set forth in the claims rather than the foregoing description, and all differences within the scope equivalent thereto should be construed as being included in the present invention.

## Claims

1. A focused ultrasound apparatus comprising:
a treatment module configured to receive information on parameters of a treatment ultrasound pulse signal (hereinafter, referred to as "treatment ultrasound parameters") and generate the treatment ultrasound pulse signal according to the received treatment ultrasound parameter information; and
an imaging module configured to receive the treatment ultrasound parameter information and information on parameters of a diagnosis ultrasound pulse signal (hereinafter, referred to as "diagnosis ultrasound parameters"), analyze the received treatment ultrasound parameter information and diagnosis ultrasound parameter information in a frequency domain, adjust the diagnosis ultrasound parameter information such that the treatment ultrasound pulse signal and the diagnosis ultrasound pulse signal do not interfere with each other, and generate the diagnosis ultrasound pulse signal according to the adjusted diagnosis ultrasound parameter information.

2. The focused ultrasound apparatus of claim 1, wherein the imaging module shifts a frequency of the diagnosis ultrasound pulse signal (hereinafter, referred to as a "diagnosis ultrasound frequency") such that a frequency of the treatment ultrasound pulse signal (hereinafter, referred to as a "treatment ultrasound frequency") and the diagnosis ultrasound frequency do not overlap each other in a frequency domain, and generates a new diagnosis ultrasound frequency by shifting the frequency.

3. The focused ultrasound apparatus of claim 2, wherein the shifted new diagnosis ultrasound frequency is greater than a sum of the treatment ultrasound frequency and a bandwidth of the diagnosis ultrasound pulse signal (hereinafter, referred to as a "diagnosis ultrasound bandwidth").

4. The focused ultrasound apparatus of claim 1, wherein the imaging module comprises a diagnosis ultrasound pulse generator configured to receive diagnosis ultrasound parameter information including information on a center frequency of a diagnosis ultrasound pulse signal (hereinafter, referred to as a "diagnosis ultrasound center frequency") (Fc_img) and information on a diagnosis ultrasound bandwidth (Fc_img B.W), receive treatment ultrasound parameter information including information on a center frequency of a treatment ultrasound pulse signal (hereinafter, referred to as a "treatment ultrasound center frequency") (Fc_fus), and adjust the diagnosis ultrasound parameter information for non-interference between the treatment ultrasound pulse signal and the diagnosis ultrasound pulse signal by using the diagnosis ultrasound parameter information and the treatment ultrasound parameter information.

5. The focused ultrasound apparatus of claim 4, wherein the diagnosis ultrasound pulse generator shifts the diagnosis ultrasound center frequency (Fc_img) to generate a new diagnosis ultrasound center frequency (Fc'_img) such that the new diagnosis ultrasound center frequency (Fc'_img) is greater than a sum of the treatment ultrasound center frequency (Fc_fus) and the diagnosis ultrasound bandwidth (Fc_img B.W) in a frequency domain.

6. The focused ultrasound apparatus of claim 1, wherein the imaging module comprises an image processing unit configured to generate a diagnosis image by receiving an ultrasound echo signal in an interval in which the frequency of the treatment ultrasound frequency and the frequency of the diagnosis ultrasound frequency) do not overlap.

7. The focused ultrasound apparatus of claim 1, wherein:
the treatment module acquires timing information regarding an interval in which the treatment ultrasound pulse signal is actually generated in a time domain, and
the imaging module receives the timing information from the treatment module and generates the diagnosis ultrasound pulse signal during an interval in which the treatment ultrasound pulse signal is not generated.

8. The focused ultrasound apparatus of claim 7, wherein the treatment module comprises:
a signal extraction unit configured to extract an active period of the treatment ultrasound pulse signal generated in each channel of a treatment ultrasound transducer; and
a summation unit configured to obtain an overall active period of the treatment ultrasound pulse signals by performing a logical OR operation on the active periods of the treatment ultrasound pulse signals of the respective channels.

9. The focused ultrasound apparatus of claim 8, wherein the signal extraction unit comprises:
an attenuator configured to attenuate the treatment ultrasound pulse signal generated in each channel; and
an envelope detector configured to extract an envelope of each of the attenuated treatment ultrasound pulse signals.

10. The focused ultrasound apparatus of claim 8, wherein:
the summation unit transmits an image pulse activation signal to the imaging module during an interval excluding an overall treatment ultrasound active period, and
the imaging module generates the diagnosis ultrasound pulse signal during an interval excluding the overall treatment ultrasound active period according to the image pulse activation signal.

11. The focused ultrasound apparatus of claim 8, further comprising:
a comparator configured to receive, as respective inputs, a signal extracted through the signal extraction unit and a reference signal, compare the extracted signal with the reference signal, and output the extracted signal as a logic signal in case that the extracted signal has a value equal to or greater than a value of the reference signal; and
a reference providing unit configured to provide the reference signal to the comparator.

12. The focused ultrasound apparatus of claim 11, wherein the reference providing unit is a digital-to-analog converter capable of varying the value of the reference signal.

13. A non-interference method between ultrasound pulse signals using a focused ultrasound apparatus, comprising:
receiving, by an imaging module of the focused ultrasound apparatus, treatment ultrasound parameter information and diagnosis ultrasound parameter information;
adjusting the diagnosis ultrasound parameter information such that a treatment ultrasound pulse signal and a diagnosis ultrasound pulse signal do not interfere with each other by analyzing the received treatment ultrasound parameter information and diagnosis ultrasound parameter information in a frequency domain;
generating the diagnosis ultrasound pulse signal according to the adjusted diagnosis ultrasound parameter information and transmitting the generated diagnosis ultrasound pulse signal to tissue; and
generating a diagnosis image using an ultrasound echo signal returned from the tissue.
